# EUROPEAN PATENT APPLICATION

(11) **EP 4 282 477 A2**
(43) Date of publication of application: **29.11.2023**
(21) Application number: 23202770.6
(22) Date of filing: 25.10.2019
(51) Int. Cl.: A61P 19/02

(54) **COMBINATION COMPRISING SILDENAFIL FOR USE IN THE TREATMENT OF OSTEOARTHRITIS**

(30) Priority: 26.10.2018 SE 1851333
(62) Divisional of application: 19801226.2
(71) Applicant: Artroa AB, 436 40 Askim (SE)
(72) Inventor: SKIÖLDEBRAND, Eva, 412 67 GÖTEBORG (SE); HANSSON RÖNNBÄCK, Elisabeth, 436 39 ASKIM (SE)
(74) Representative: Noréns Patentbyrå AB

(57) **Abstract**

There is provided a new treatment for osteoarthritis, which involves the administration of a phosphodiesterase type 5 (PDES) inhibitor, for example sildenafil in a low dose, to the patient.

## Description

### Field of the invention

This invention is related to novel treatments of osteoarthritis, in particular osteoarthritis in humans and horses.

### Background

Osteoarthritis (OA) is a low-grade chronic systemic inflammatory disease that results from breakdown of joint cartilage and underlying bone. The disease proceeds from an early phase which often is characterized by inflammation and disorganization of matrix proteins in the affected joints, to a more severe phase with damage to the underlying bone. The main symptom is pain in the joint. Osteoarthritis is a major clinical problem as it affects around 3.8% of the human population, and it is the major disease behind severe joint pain. It is estimated that 50% of NSAID pain killer prescriptions are related to OA. It should be noted that osteoarthritis is a completely different disease from rheumatoid arthritis, which is a rare autoimmune disease.

Osteoarthritis in horses is a great problem for horse owners. The principal reason for having a horse is to be able to use it, and often to compete with it. OA is the most frequent reason for not being able to use horses and accounts for the greatest single economic loss in the horse industry.

OA in horses and humans are caused by similar mechanisms. In both humans and horses OA proceeds from an early stage characterized by inflammation over months and years to a later stage with extensive tissue damage (Goldring, M.B., Otero M. Current Opinion in Rheumatology (2011) 23(5):471). OA disease mechanisms in humans and horses are also very similar on the molecular level (Stenberg J, Rüetschi U, Skiöldebrand E, Kärrholm J, Lindahl A. Proteome Sci. (2013) Oct 4;11(1):43) (Svala E, Löfgren M, Sihlbom C, Rüetschi U, Lindahl A, Ekman S, Skiöldebrand E. Connect Tissue Res. (2015);56(4):315-25). Intracellular calcium signalling is increased in inflammatory chondrocytes (Skiöldebrand E, Thorfe A, Björklund U, Johansson P, Wickelgren R, Lindahl A, Hansson E. Heliyon. (2018) Feb 1;4(1):e00525).

Treatment of OA in humans is today limited to changes in life style (such as weight loss and exercise) and the use of analgesics, and in severe cases, joint replacement surgery. Joint injection of glucocorticoids (such as hydrocortisone) leads to short term pain relief that may last between a few weeks and a few months.

OA in horses may be treated with for example injection of glucocorticoids, hyaluronic acid or autologous conditioned serum (ACS).

However, no disease-modifying drugs exists, for humans or horses. Thus, there is a need for improved treatment of OA.

This invention solves these and other problems.

### Summary of invention

The inventors have surprisingly found that an inhibitor of phosphodiesterase type 5 (PDE5), such as sildenafil or a sildenafil analogue, in particular at low concentrations, can be used in the treatment of OA. Sildenafil (CAS number 139755-83-2) is typically used for treating erectile dysfunction and is sold under the trademark Viagra^{®}. Surprisingly, low doses of sildenafil and sildenafil analogues are capable of inhibiting intracellular calcium signalling, in particular ATP-evoked intracellular calcium signalling. Without being bound by any theory it is believed that sildenafil is able to achieve a balance between intercellular calcium signalling through gap junctions and extracellular signalling mediated by extracellular ATP. The ATP-evoked calcium signalling is important due to increased ATP release and subsequent activation of purinergic receptors.

In a first aspect of the invention there is provided a phosphodiesterase type 5 (PDE5) inhibitor or a pharmaceutical composition that comprises a PDE5 inhibitor for use in the treatment of osteoarthritis. The PDE5 inhibitor may be sildenafil or an analogue to silden-fil. The dose of sildenafil may be a low dose, for example from 0.01 mg to 10 mg, or more preferred 0.01 mg to 0.1 mg. The dose is preferably administered at least once, more preferably at least twice. The dose may be administered one, two or three times. In a preferred embodiment the dose is administered once, in an even more preferred embodiment two times. In one embodiment the PDE5 inhibitor is be injected into the affected joint, in particular one, two or three times.

In one embodiment, the PDE5 inhibitor or the sildenafil analogue is administered together with at least one compound selected from the group consisting of: a local anaesthetic and glucose, preferably both a local anaesthetic and glucose. In certain embodiments, glucose may be replaced with metformin. Thus, the PDE5 inhibitor may be administered together with metformin.

In a preferred embodiment the PDE5 inhibitor is sildenafil and the local anaesthetic is mepivacaine and the dose of sildenafil is from 0.01 mg to 0.1 mg, the dose of glucose is from 100 mg to 1000 mg and the dose of mepivacaine is from 10 mg to 200 mg. In one embodiment the dose of glucose is from 150 mg to 400 mg and the dose of mepivacaine is from 10 mg to 30 mg.

In a second aspect of the invention there is provided a pharmaceutical composition comprising a PDE5 inhibitor or a sildenafil analogue and one of glucose and a local anaesthetic, preferably both glucose and a local anaesthetic, or a PDE5 inhibitor, metformin and a local anaesthetic. The pharmaceutical composition may be an injection solution.

In a third aspect of the invention there is provided a method for treatment of osteoarthritis in a subject comprising administering a PDE5 inhibitor, such as sildenafil or a sildenafil analogue or a pharmaceutical composition comprising a PDE5 inhibitor such as sildenafil or a sildenafil analogue to said subject. The method may comprise the step of injecting the PDE5 inhibitor, or the pharmaceutical composition comprising a PDE5 inhibitor, into the affected joint. The PDE5 inhibitor may be sildenafil, in particular sildenafil in a dose from 0.01 mg to 10 mg.

There is also provided the use of a PDE5 inhibitor or a sildenafil analogue for the manufacture of a medicament for the treatment of osteoarthritis.

### Figures

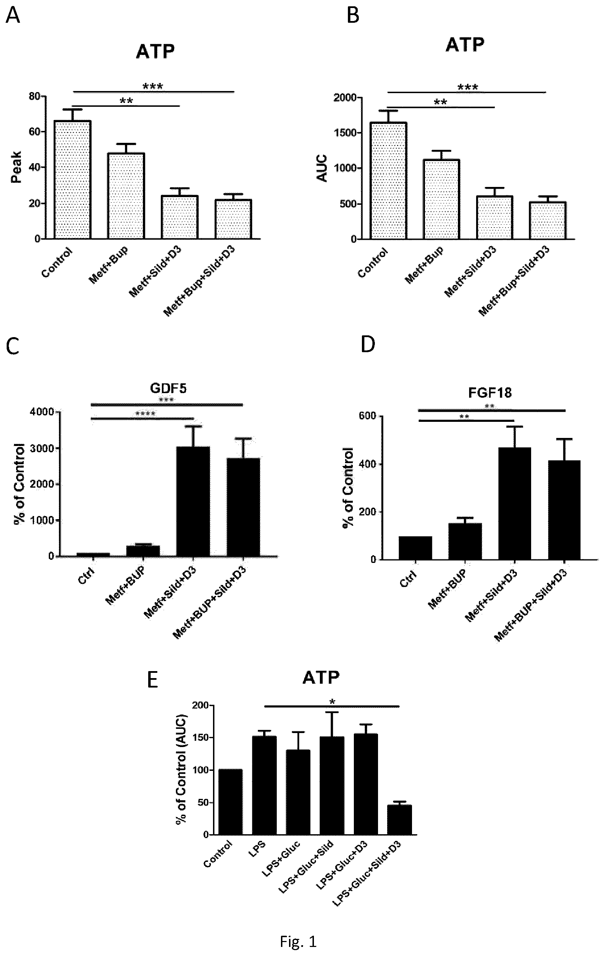

Figs. 1A-E are graphs showing effects of treatment of bupivacaine and sildenafil on chondrocytes.

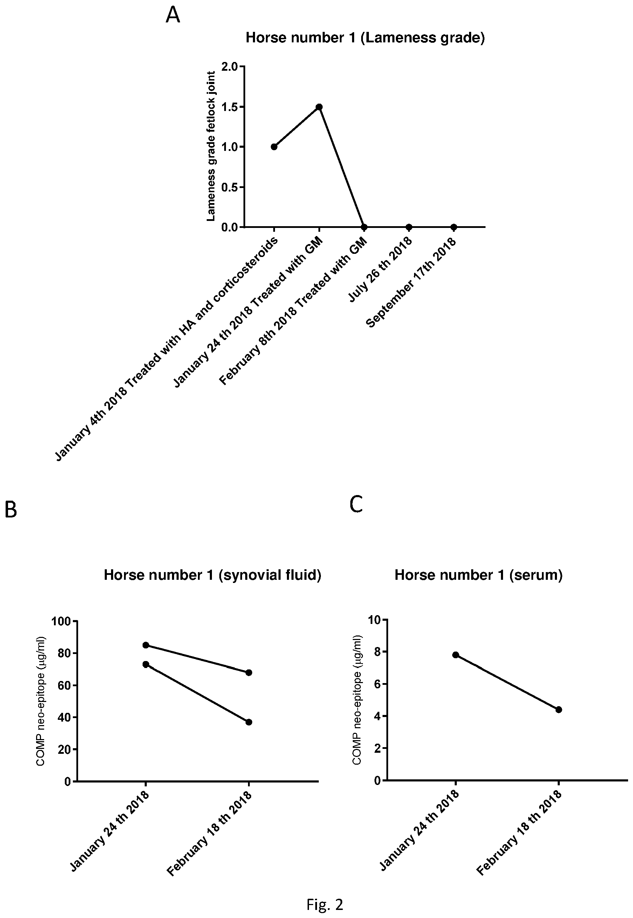

Fig. 2 shows the effect of low dose mepivacaine and glucose treatment of a horse with OA. Fig. 2A shows clinical lameness grade of carpal joints after treatment with glucose/mepivacaine. Fig. 2B is a graph that shows decreased concentration of COMP neo-epitope in synovial fluid after treatment with glucose/mepivacaine (GM). Fig. 2C is a graph that shows decreased concentration of COMP neo-epitope in serum after treatment with glucose/mepivacaine and during rehabilitation.

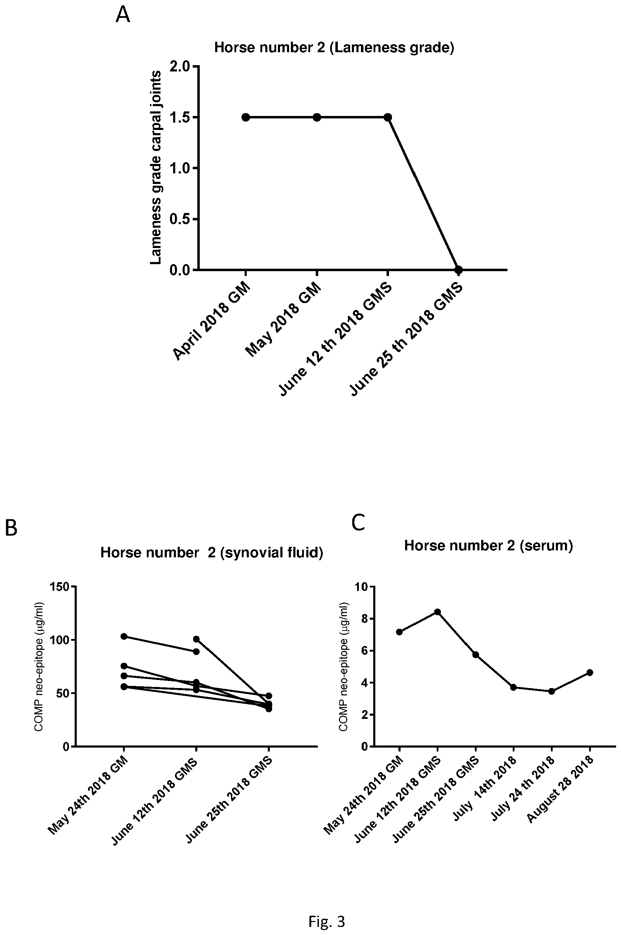

Fig. 3 shows the effect of sildenafil treatment of a horse with OA. Fig. 3A shows clinical lameness grade of carpal joints after treatment with glucose/mepivacaine/sildenafil (GMS). Fig. 3B is a graph that shows decreased concentration of COMP neo-epitope in synovial fluid after treatment with glucose/mepivacaine/sildenafil (GMS). Fig. 3C is a graph that shows decreased concentration of COMP neo-epitope in serum after treatment with glucose/ mepivacaine /sildenafil (GMS) and during rehabilitation.

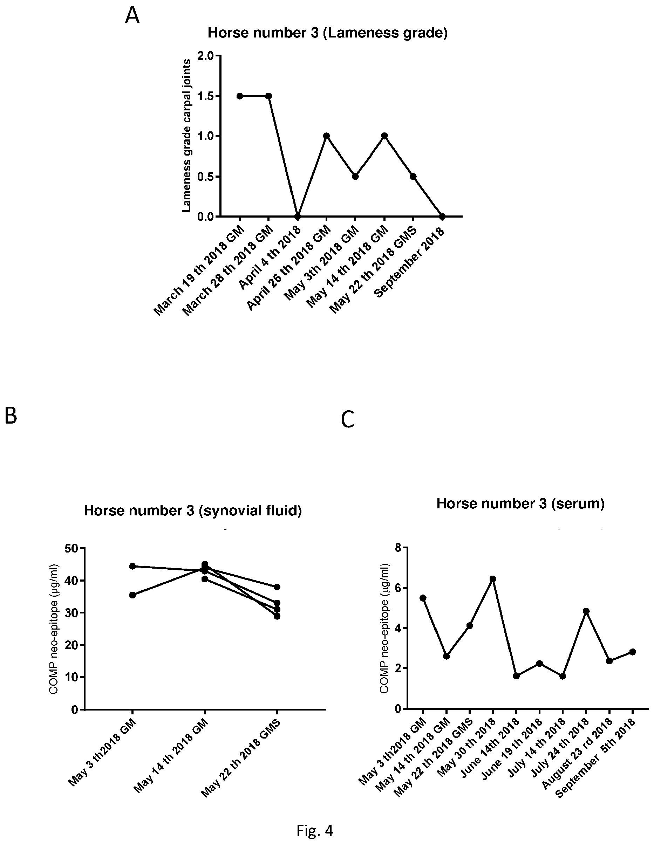

Fig. 4 shows the effect of sildenafil treatment of a horse with OA. Fig. 4A is a graph that shows clinical lameness grade of carpal joints before and after treatment with glucose/mepivacaine/sildenafil (GMS). Fig. 4B is a graph that shows decreased concentration of COMP neo-epitope in synovial fluid after treatment with glucose/mepivacaine /sildena-fil (GMS). Fig. 4C is a graph that shows decreased concentration of COMP neo-epitope in serum after treatment with glucose/ mepivacaine /sildenafil (GMS) and during rehabilitation.

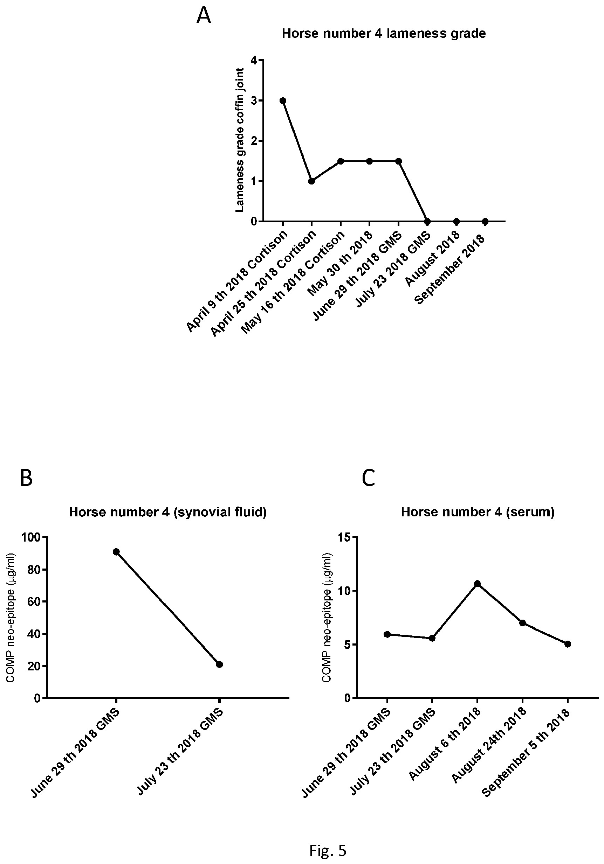

Fig. 5 shows the effect of sildenafil treatment of a horse with OA. Fig. 5A is graph that shows clinical lameness grade before and after treatment with corticosteroids or glucose/mepivacaine/sildenafil (GMS). Fig. 5B is graph that shows decreased concentration of COMP neo-epitope in synovial fluid after treatment with glucose/ mepivacaine /sildenafil (GMS). Fig. 5C is a graph that shows concentration of COMP neo-epitope in serum before and after treatment with glucose/mepivacaine/sildenafil (GMS) and during rehabilitation.

### Detailed description

There is provided a treatment of osteoarthritis, in particular a treatment by reducing the inflammation in the joint, in particular reducing inflammation signalling, such as calcium signalling, within and between chondrocytes in the joint. The treatment preferably has at least one effect of: reducing pain, inducing healing of the joint, re-growth of matrix, and decreased inflammation.

Diagnosis of OA may be done with methods known in the art, for example clinical examination, radiography as is known in the art or the use of biomarkers, such as, for example, the COMP protein fragment described in WO2017216289, which is incorporated herein by reference. The presence of the COMP fragment in a bodily fluid, for example serum or synovial fluid, indicates OA, in particular cartilage degradation during OA. The COMP fragment may be detected by using an antibody that specifically binds to a peptide comprising the amino acid sequence N-terminal-SGPTH. It is referred to WO2017216289 for details.

Thus, in one embodiment, the patient is treated as described herein and the presence of a fragment of COMP is detected. The detection of the COMP fragment may be used to follow how the patient responds to the treatment.

The patient may be a human or an animal. When the patient is an animal it may preferably be a horse, but many different kinds of animals may benefit from treatment, including dogs, cats and cattle.

There is provided the use of a PDE5 inhibitor, in particular sildenafil, or a sildenafil analogue, for the treatment of osteoarthritis. The PDE5 inhibitor may be selected from the group consisting of the approved drugs sildenafil, avanafil, lodenafil, mirodenafil, tadalafil, vardenafil and udenafil, where sildenafil is preferred. Modified variants of these substances as well as analogues of these substances may also be used (see below). Examples of compounds that are currently not approved as drugs, but which display PDE5 inhibitory activity include: hongdenafil, aildenafil, benzamidenafil, homosildenafil, icariin, nitrosoprodenafil, sulfoaildenafil and zaprinast.

PDE5 is an enzyme that catalyses the break-down on cGMP, which is an important second messenger in the cell. The PDE5 inhibitor may compete with cGMP for binding to the active site of PDE5. Preferably the PDE5 inhibitor is a molecule that interacts directly with the PDE5 protein, preferably by binding to the cGMP-binding pocket of PDE5. The PDE5 inhibitor may be a substance that has higher affinity for PDE5 than cGMP. The PDE5 inhibitor is preferably a selective PDE5 inhibitor.

PDE5 inhibitors may be identified by their capacity to inhibit PDE5 in vitro. For example, in a cell-based assay, using cells that express PDE5, or by using purified PDE5 as is known in the art. A library of compounds may be screened for their ability to inhibit PDE5. Examples of compounds that may be screened include the compounds disclosed in WO9428902. Further compounds having PDE5 phosphodiesterase-inhibiting activity are described for example in j. Med. Chem. 1993, 36:3765 ff. and in j. Med. Chem. 1994, 37:2106 ff. The structure of cGMP is also a suitable starting point for the design of a PDE5 inhibitor.

In one embodiment, a PDE5 inhibitor, in particular sildenafil, is used as a single agent, i.e. as the single active compound in a pharmaceutical composition. Excipients are not considered to be active compounds.

A sildenafil analogue is a compound with a structure similar to sildenafil and which is capable of reducing intracellular calcium signalling in cells in vitro, in particular in chondrocytes, in particular ATP-evoked calcium signalling. As used herein, "intracellular calcium signalling" refers to the release of calcium ions in the cytosol from the endoplasmic reticulum. The intracellular calcium signalling may be ATP-evoked, meaning that it is caused by the addition of ATP to the cell growth medium of suitable cells that are connected by gap junctions. Such addition of ATP will cause waves of intracellular calcium release from the endoplasmic reticulum in the cells. Hence, a sildenafil analogue is able to inhibit such calcium signalling. Preferably the inhibition can be determined in vitro, such as in cell culture.

Sildenafil analogues may or may not be PDE5 inhibitors, hence they may or may not have the ability to inhibit PDE5 signalling. Sildenafil analogues may be identified by their capability to decrease calcium signalling in vitro, in particular ATP-evoked calcium signalling in cells cultured in vitro, for example in chondrocytes. Calcium signalling may be assessed using any suitable method. Methods include high-throughput screening systems for intracellular calcium signalling, for example Flexstation 3 Microplate Reader, where the cells are incubated with a calcium-sensitive probe; calcium imaging systems with, for example, Simple PCI software and an inverted epifluorescence microscope, where the cells are incubated with a calcium-sensitive probe; a SPEX Fluoromax imaging system with adopted software, an inverted epifluorescence microscope, and a calcium-sensitive probes; a PTI imaging system with adopted software, an inverted epifluorescence microscope, and a calcium-sensitive probe. Examples of useful calcium sensitive probes include Fluo-3/AM or Fura-2/AM (Molecular Probes, Eugene, Oregon, USA.

The reduction of calcium signalling by the sildenafil analogue may be 50% or more, 70% or more, or higher, such as 80% or more.

A suitable protocol for testing for the ability of a compound to inhibit ATP-evoked calcium signalling is the following: Astrocytes or chondrocytes are grown to confluence in DMEM so that they become gap-junction coupled in a monolayer. Cells are then incubated at room temperature with the Ca2+-sensitive fluorophore Fura-2/AM (Molecular Probes, Eugene, OR, USA) for 30 min (8 µl in 990 µl Hank's HEPES buffered saline solution (HHBSS), consisting of 137 mM NaCl, 5.4 mM KCI, 0.4 mM MgSO4, 0.4 mM MgCl2, 1.26 mMCaCl2, 0.64 mM KH2PO4, 3.0 mM NaHCO3, 5.5 mM glucose, and 20 mM HEPES dissolved in distilled water, pH 7.4). All substances used during the experiment are diluted in the same solution. The fluorophore was dissolved in 40 µl of dimethyl sulfoxide (DMSO) and 10 µl of pluronic acid (Molecular Probes, Leiden, The Netherlands). After incubation, the cells are rinsed three times with HHBSS. After one minute from the beginning of the experiment ATP (10-4M) is used as the stimulator. The experiments are performed at room temperature using a Ca2+imaging system and simple software PCI (Compix Inc., Imaging Systems, Hamamatsu Photonics Management Corporation, Cranberry Twp, PA, USA) and an inverted epifluorescence microscope (Nikon ECLIPSE TE2000-E). The ATP is provided by a peristaltic pump (Instech Laboratories, Plymouth Meeting, PA, USA) at a flow rate of 600 µl/min. The images are captured with a ORCA-12AG camera (C4742-80-12AG), High Res Digital cooled CCD (Hamamatsu Photonics Corporation, Hamamatsu, Japan). The total areas under curve (AUC) is then determined, reflecting the amounts of Ca2+released is analysed to provide measurements of Ca2+ responses. The AUC and the amplitude (peak) is calculated using the Origin program (Microcal Soft-ware Inc., Northampton, MA, USA). The candidate substance is added at a suitable concentration and tested for ability to decrease the ATP-induced intracellular calcium signalling.

As an alternative the following equipment can be used: With a high-throughput screening system for intracellular Ca2+ signalling, Flexstation 3 Microplate Reader (Molecular Devices, San José, USA), the cells are incubated with the Ca2+-sensitive probe FLIPR Calcium 6 (Molecular Devices) and stimulated immediately before the experiment started with ATP (10- 4 M) (Sigma Aldrich Saint Louis, USA). The intracellular Ca2+ release is determined as the total areas under the curve (AUC), which reflects the amount of Ca2+ released. The amplitude (peak) was expressed as the maximum increase (Hansson E, Björklund U, Skiöldebrand E, Rönnbäck L. J Neuroinflammation (2018); 15:321. https://doi.org/10.1186/s12974-018-1361-8.

Sildenafil analogues are structurally similar to sildenafil. Structurally similar compounds are compounds that have four, three, two or preferably one substitution in relation to the molecular structure of sildenafil. Possible substitutions include replacement of groups with other groups or addition of group in relation to the structure of sildenafil. "groups" may refer to, for example a methyl group or other alkanes, a halogen atom, an amide or an amine, or any other type of useful group used in the art of medicinal chemistry. Whereas there are many possible compounds that are structurally similar to sildenafil, it is straightforward to verify that they are able to reduce intracellular calcium signalling in vitro using the methods described herein. Many PDE5 inhibitors, such as for example vardenafil are structurally similar to sildenafil. Below are shown the molecular structure of sildenafil and vardenafil, showing how they differ only in that in vardenafil, a nitrogen atom has been replaced with a carbon atom.

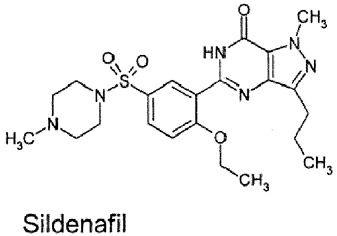

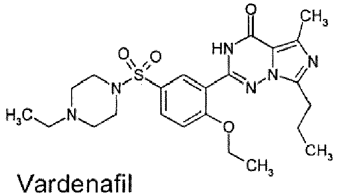

Suitable sildenafil analogues may include, avanafil, lodenafil, mirodenafil, tadalafil, vardenafil and udenafil, hongdenafil, aildenafil, homosildenafil, nitrosoprodenafil, sulfoaildenafil and zaprinast.

### Routes of administration

The PDE5 inhibitor or the sildenafil analogue may be administered in any suitable manner, including orally, topically, intravenously, intradermally, or intraarticularly, intramuscular, nasal, or into the cerebrospinal fluid.

In a preferred embodiment the PDE 5 inhibitor or the sildenafil analogue is delivered locally to the affected joint. In an even more preferred embodiment, the formulation is delivered intraarticularly, in particular with one or more intraarticular injections into the affected joint, i.e. injection into the joint.

The PDE5 inhibitor or the sildenafil analogue may be administered by topical administration by, for example, transdermal administration. The transdermal formulation is specifically advantageous in regard of simplicity and from a patient comfort standpoint. It may, for instance, take the form of a transdermal patch.

In one embodiment, the PDE5 inhibitor or the sildenafil analogue is delivered so that at least some systemic distribution of the PDE5 inhibitor is achieved. This may be achieved local administration where the drug spreads through the body or with systemic administration, for example oral administration or intravenous administration.

In addition to the methods of administration mentioned above also parenteral, intranasal, and rectal administration may be useful, as well as administration by inhalation.

The timing of the administration of the PDE5 inhibitor or the sildenafil analogue according to the invention will depend on the formulation and/or route of administration used.

When the PDE5 inhibitor or the sildenafil analogue is administered with local injection, one or more injections may be carried out. A suitable number of injections may be one, two, three, or more. The plurality of injections may be carried out with an interval of 2- 60 days, more preferably 5-30 days, even more preferably 8-25 days and most preferably 10-18 days. The recovery of the joint may be monitored using the measurement of the COMP fragment as described in WO2017216289, for example by monitoring the presence of the COMP fragment in serum or in synovial fluid. Other methods known in the art may be used, for example radiology.

In general, a pharmacologically effective amount of a PDE5 inhibitor or a sildenafil analogue is provided to a patient in need thereof. The appropriate dose range for the compound can be established in routine experiments. The dose may be lower than the sildenafil dose used to treat erectile dysfunction (which is often about 100 mg). The dose per administration of sildenafil given to a horse may be from 0.001 mg to 1000 mg, in particular 0.01 mg to 10 mg, in particular 0.01 mg to 1 mg, in particular 0.01 mg to 0.1 mg and most preferably 0.01 mg to 0.05 mg or 0.01 mg to 0.03 mg. Accordingly, the dose may be at least 0.001 mg, or at least 0.01 mg, or at least 0.1 mg, or at least 1 mg, or at least 10 mg. The dose given to human is approximately the same, in particular in the case of local administration, for example when intra-articular injection is used. Alternatively, a lower dose, which is approximately a fifth of the dose given to the horse, is given to the human.

The dose may be administered once but may be administered a suitable number of times, such as once, two times, three times, or more. The dose may be administered at least once or more preferably at least twice. In a preferred embodiment the dose is administered once, in an even more preferred embodiment two times.

The PDE5 inhibitor or the sildenafil analogue may be combined with one or more other agents for treatment of osteoarthritis. Preferably the PDE5 inhibitor or the sildenafil analogue is combined with a local anaesthetic or glucose or both. The local anaesthetic may be a compound that binds to the intracellular portion of voltage-gated sodium channels and blocks sodium influx into nerve cells. Examples include bupivacaine, mepivacaine, lidocaine, prilocaine, ropivacaine, chloroprocaine and articaine. The local anaesthetic may be a local anaesthetic of the amino amide type or amino ester type. In a preferred embodiment both a local anaesthetic and glucose is included in the formulation.

The dose of the local anaesthetic may be a low dose. The dose may be so low that the normal inhibition of the voltage gated channel associated with the local anaesthetic is not achieved. When mepivacaine is used, the dose administered at one time may be from 1 mg to 1000 mg, in particular from 5 mg to 200 mg, even more preferred from 8 mg to 100 mg and most preferred from 10 mg to 30 mg. The dose of glucose administered at one time may be from 10 mg to 10 000 mg, in particular from 100 mg to 1000 mg, where 150 mg to 400 mg is an even more preferred range.

In one embodiment, a low dose of a local anaesthetic, for example mepivacaine, is administered together with glucose (or metformin) but without a PDE5 inhibitor or sildenafil analogue. Administration is preferably by injection, even more preferably by intra articular injection as described herein. The dose of the local anaesthetic may be from 1 mg to 1000 mg, in particular from 5 mg to 200 mg, even more preferred from 8 mg to 100 mg and most preferred from 10 mg to 30 mg. A pharmaceutical composition may thus comprise only a local anaesthetic and glucose. Injections may be carried out one, two, three or more times, with from 5 to 30 days, more preferably from 10 to 18 days, between each injection. This treatment may be preferred for patients, in particular horses, with early stage osteoarthritis.

In one embodiment, a PDE5 inhibitor or a sildenafil analogue, preferably sildenafil, is administered by intraarticular injection in a dose which preferably is from 0.01 mg to 0.1 mg together with a local anaesthetic, preferably mepivacaine, preferably in a dose of from 10 mg to 200 mg, together with glucose, preferably in a dose of from 100 mg to 1000 mg. The dose may be administered once but may be administered a plurality of times, for example 2, 3, 4 or 5 times or more, where 2 or 3 times are preferred, and 2 times is most preferred. In a preferred embodiment injection is carried out 1, 2 or 3 times. The dose may be administered at least once or more preferably at least twice.

When multiple doses are given a suitable dose interval is 2-60 days, more preferably 5-30 days, even more preferably 8-25 days and most preferably from 10 to 18 days.

Recovery from OA after and during treatment may be monitored by measuring the presence of the COMP fragment, or other means, as described above.

Intraarticular injection is done as is known in the art. Injection may be done with any suitable technology, for example a disposable syringe and needle, an autoinjector, an injection pen or an infusion pump. Examples of needles useful for horse intraarticular injections are 0.90x40 mm or 70 mm (20G×1½"), 0.80x40mm, (21G × 1½") and spinal needle 1,20 mm × 152/200mm (18Gx6.00") for injection into very large joints.

An appropriate volume of injection solution is injected, where the volume is typically larger for a horse than for a human.

### Pharmaceutical composition

The PDE5 inhibitor or the sildenafil analogue may be comprised in a suitable pharmaceutical composition. In general, the composition should be adapted to the manner of administration and various compositions comprising a PDE5 inhibitor or the sildenafil analogue are known in the art. The composition suitably contains a therapeutically effective amount of the PDE5 inhibitor together with a suitable amount of a carrier or vehicle, in order to provide a form appropriate for the administration to the patient. The composition may have a solid, semisolid, gelatinous or hydrogel-like, a liquid and may be in the form of a tablet, a liquid, an injection solution, a suspension, a powder, a capsule, a sustained release form, a dermal patch, a gel, a cream, an ointment, a spray, or an aerosol.

A composition may, for example, comprise one or more pharmacologically acceptable excipients, including carriers, diluents, stabilizers, fillers, disintegrants, preservatives, gelling or thickening agents, release-control agents as is known in the art. Examples of compounds that may be used in the composition include: starch, cellulose and cellulose derivatives, gelatine, silica gel, magnesium carbonate, magnesium stearate, sodium stearate, glycerol monostearate, talc, sodium chloride, glycerol, glycol, water, and ethanol.

Peroral formulations of sildenafil are known in the art, for example the formulation of Viagra^{®}. For preparing further preparations for peroral administration reference is made to Pharmaceutical Dosage Forms: Tablets. Vol. 1-3, H A Lieberman et al., Eds. Marcel Dekker, New York and Basel, 1989-1990. In particular specific reference is made to chapter 7 (Special Tablets, by J W Conine and M J Pikal), chapter 8 (Chewable Tablets, by R W Mendes, OA Anaebonam and J B Daruwala), and chapter 9 (Medicated Lozenges; by D Peters). The PDE5 inhibitor may be provided as a pharmaceutically acceptable salt. Sildenafil may for example be provided as sildenafil citrate.

Injection solutions comprising sildenafil are also known in the art. One example of an injection solution comprising sildenafil is Revatio^{®}. The injection solution is preferable an aqueous solution. The injection solution may comprise one or more of the following pharmacologically acceptable agents: a PH-adjusting agent, saline, osmolarity-controlling agents, stabilizers, preservatives, chelating agents, solubilizing agents, antibacterial agents and emulsifying agents. The injection solution may, for example, contain only a PDE5 inhibitor, for example sildenafil, and pharmacologically grade water, and, optionally, a preservative. The injection solution may also comprise one or both of a local anaesthetic and glucose.

The injection solution is preferably sterile and isotonic. The injectable solution may be provided in a container such as a vial, such as an injection vial, a sachet or a cartridge. A solution for injection may be provided in an injection device for single use such as an auto injection device or an injection pen. The solution for injection may be premixed or in a form to be reconstituted, for example by addition of pharmacologically acceptable water.

When the formulation is a formulation for local delivery, for example by injection, it may also preferably comprise one of glucose and a local anaesthetic.

In one embodiment mentioned above, the formulation comprises only glucose and a local anaesthetic. When a formulation only comprises a local anaesthetic and glucose as active agents, the formulation is preferably an injection solution.

The preferred concentration of sildenafil or the sildenafil analogue in the injection solution is from 0.001 mg/ml to 0.1 mg/ml, more preferably 0.001 - to 0.01 mg/ml, in particular where a low dose of sildenafil is administered. The preferred concentration of local anaesthetic, for example mepivacaine, is from 0.1 mg/ml to 50mg/ml, more preferably from 1 mg/ml to 5 mg/ml. The preferred concentration of glucose is from 1 mg/ml to 200 mg/ml, more preferably from 10 mg/ml to 100 mg/ml. Where higher doses are administered, concentrations of these substances may be higher. The concentration ratio of sildenafil to mepivacaine to glucose in the formulation is preferably 0.001:1:10.

Formulations comprising sildenafil are described in WO 02/060449 and WO 02/062343. Synthesis of sildenafil is described in EP463756 and US5250534. Synthesis of Avanafil is described in US patent 7,501,409.

### Example 1 - decrease of inflammation in isolated chondrocytes

In vitro experiments suggested that treatment of horse chondrocytes with low concentration of local anaesthetic bupivacaine decreased intracellular calcium signalling and upregulated growth factors of importance for generation of cartilage. The addition of sildenafil in low concentration increased the effect.

Horse chondrocytes from OA horses were stimulated, in a fluorescence-based assay for detecting changes in intracellular Ca²⁺ over time, with ATP (10⁻⁴ M). The cells were cultivated in 5.5 mM glucose the whole cultivation period. Ca²⁺ responses were measured after the cells were incubated for 24 h with metformin (1 mM), and bupivacaine (10⁻¹² M). Controls are untreated chondrocytes. The area under the Ca²⁺ peak (AUC)(Fig 1 A) was calculated for each Ca²⁺ transient as well as the amplitude (peak) (Fig. 1 B) was expressed as the maximum increase. The cells were obtained from 4 experiments with quadruple wells in each. The level of significance was analyzed using a one-way ANOVA followed by Dun-nett's multiple comparisons test. ** *P*< 0.01, ** *P*< 0.001. Similar data was obtained for human chondrocytes.

### Materials and Methods

### Cell model system - Chondrocyte isolation and culture

Articular cartilage samples were obtained, within 24 h of euthanasia, from the middle carpal joints of six horses (1- 8-year-old). Three horses had a normal macroscopically joint surface and three horses had a mild structural OA in the radial facet of the middle carpal bone. The horses were euthanized because of reasons unrelated to this study, and the Ethical Committee on Animal Experiments, Stockholm, Sweden, approved the study protocol (Dnr: N378/12).

Following aseptic preparation, cartilage of the dorsal aspect of the radial facet of the third carpal bone was incised with a scalpel, and full-thickness cartilage samples were collected with forceps. The tissue was placed in a sterile saline (0.9% NaCl) solution with gentamicin sulfate (50 mg/l) and amphotericin B (250 µg/ml). Cartilage samples were transported chilled (approx. 5°C) to the laboratory. Isolation and expansion of chondrocytes were performed as previously described (Ley C, Svala E, Nilton A, Lindahl A, Eloranta M, Ekman S, Skiöldebrand E.Connect Tissue Res. (2011); 52 (4): 290-300). Cells were expanded in passage 1 and cells were seeded at 20,000 cells/cm2 in chondrogenic media to maintain the phenotype, DMEM-high glucose (DMEM-HG) (Thermo Fisher Scientific; Waltham, MA, USA) supplemented with 14 µg/ml ascorbic acid (Sigma-Aldrich, St. Louis, MO, USA), 10⁻⁷ M dexamethasone (Sigma-Aldrich), 1 mg/ml human serum albumin (Equitech Bio, Kerville, TX, USA), 1 × insulin-transferrin-selenium (Gibco, Life Technologies, Carlsbad, CA, USA), 5 µg/ml linoleic acid (Sigma-Aldrich), 1 × penicillin-streptomycin (PEST) (Sigma-Aldrich), and 10 ng/ml human transforming growth factor (TGF) β-1 (R&D Systems, Abingdon, UK) for 5 days until confluence, in 96-well culture plates for Ca²⁺or in 6-well culture plates for Western blot analysis.

For restoration the cells were incubated with metformin and bupivacaine at day 4 of cultivation for 24 h. At day 5 the Ca²⁺ measurements were performed, medium collected for Western blot analysis and cells were harvested and immediately frozen at -80°C before analysis.

For anti-inflammatory restoration the cells were incubated with different combinations of pharmacological substances for 24 h.

### Drug restoration

Restoration proceeds so that the cells are incubated with the drug substances 1 mM metformin (Sigma Aldrich, St. Louis, MO, USA) for 24 h. Drug substances are: bupivacaine (Marcain) (Astra Zeneca, Södertälje, Sweden) (10⁻¹² M) (Block L, Jörneberg P, Björklund U, Westerlund A, Biber B, Hansson E. Eur. J. Neurosci. (2013); 38: 3669-3678), sildenafil citrate salt (Sigma Aldrich) (1 µM) (Nunes AKS, Raposo C, Björklund U, Cruz-Höfling MA, Peixoto CA, Hansson E, Neurosci. Lett. (2016); 630: 59-65) and 1α,25-dihydroxyvitamin D3 (calcitriol) (Sigma-Aldrich) (100 nM) (Li F, Zhang A, Shi Y, Ma Y, Du Y. Int. J. Mol. Med. (2015); 36: 967-974). The cell cultures were incubated with the above substances for 24 h before the respective experiments started.

### Calcium imaging

With a high through-put screening system for intra- and extracellular Ca²⁺-signaling, Flexstation 3 Microplate Reader (Molecular Devices, San José, USA) the cells were incubated with the Ca²⁺ sensitive probe FLIPR Calcium 6 (Molecular Devices) and exposed with different neurotransmitters; 5-HT (10⁻⁵ M), or ATP (10⁻⁴ M) all from Sigma Aldrich (Saint Louis, USA).

### Harvest of monolayer chondrocytes for RNA analysis

Cells (human chondrocytes) aimed for RNA isolation were washed with sterile PBS twice before addition of 1M DL-Dithiothreitol solution (Sigma Aldrich) in lysis buffer (Qiagen).

The cells were visually inspected to ensure lysis before transfer to RNAse free, endtoxin-free sample tubes, snap frozen in liquid nitrogen, and stored at - 80°C for RNA isolation. Total RNA was extracted and purified with a commercially available kit (RNeasy mini kit, Qiagen) in accordance with the manufacturer's protocol. In brief, cells in lysis buffer were thawed and vortexed for 1 minute before 70% ethanol was added (1:1). The samples were mixed before addition to a spin column, and centrifuged. The column was then washed before genomic DNA was removed with DNase (Qiagen). The washing step was repeated with a stringent wash buffer and thereafter a mild buffer for washing membrane bound RNA. Total RNA was eluted with 50 µl RNase free water. RNA concentration was measured with a micro-volume spectrophotometer (ND-1000 spectrophotometer, NanoDrop Technologies, Wilmington, DE) and RNA quality was evaluated using the Agilent 2100 Bioana-lyzer system (Agilent Technologies Inc, Palo Alto, CA).

### Quantitative Real-Time Polymerase Chain Reaction Analysis

cDNA was prepared from total RNA using High Capacity cDNA Reverse Transcription Kit (Applied Biosystems, Foster City, CA, USA). Commercially available TaqMan Gene Expression Assays all from Applied Biosystems were used (Fibroblast growth factor 18 (FGF-18) and Growth and differentiation factor-5 (GDF-5)). Samples were analyzed in duplicates. The relative comparative CT method was used to analyze the real-time polymerase chain reaction (PCR) data.

The total areas under the curve (AUC), which reflects the amount of Ca²⁺ released (Ber-ridge MJ. Biochem. Soc. Symp. (2007); 74: 1-7), were analysed to measure the Ca²⁺ responses. The amplitude (peak) was expressed as the maximum increase.

In conclusion, the data shown in Figs. 1A-B show that bupivacaine in low concentration together with metformin reduced intracellular Ca²⁺ release in inflammatory osteoarthritic chondrocytes to a physiological level. Sildenafil increased the effect. D3 by itself did not have an effect (Fig 1E).

Figs. 1C-D show how treatment of bupivacaine and sildenafil upregulated growth factors GDF5 and FGF18 that are of importance for generation of in human chondrocytes.

### Example 2

An injection solution was prepared as follows:
1 ml mepivacaine, 20 mg/ml =20 mg
4 ml glucose 50 mg/ml = 200 mg
5 ml sterile H2O

Final concentration of mepivacaine was 2 mg/ml. Final concentration of glucose was 20 mg/ml.
10 ml was used as the dose administered to horses 1-3 by intraarticular injection in examples 4-6, below (indicated with GM in Figs. 2-4).

### Example 3

An injection solution was prepared as follows:
Revatio^{®}(sildenafil at 0.8 mg/ml) was diluted 200 times in sterile water to obtain 0.004 mg/ml. 5 ml of this solution (0.02 mg sildenafil) was mixed with
1 ml mepivacaine, 20 mg/ml =20 mg glucose.
4 ml glucose 50 mg/ml = 200 mg glucose.
To obtain 10 ml.

Final concentrations were: sildenafil: 0.002 mg/ml, mepivacaine: 2 mg/ml, glucose: 20 mg/ml.

10 ml (sildenafil: 0.02 mg, mepivacaine: 20 mg and glucose 200 mg) was used as the dose given to horses 2-4 in examples 4-6 by intraarticular injection (indicated with GMS in Figs. 2-5).

### Example 4 - Horse number 1

Horse number 1 is a Swedish warmblood horse, mare, born 2006. It is a riding school horse that was diagnosed with bilateral lameness in both fetlock joints in January 4^{th}, 2018. The horse was treated with hyaluronic acid (HA) intra-articularly, and corticosteroids orally. The lameness was increased after second visit and the horse was then treated with a 10 ml intra-articular injection of glucose/mepivacaine (GM) of Example 1. Upon re-examination, the horse was non-lame, but was nevertheless treated once more with the same type of injection. The horse is non-lame since the first treatment with GM and has been completely non-lame at all re-examinations. The horse is back in work at the riding school in and is in full condition.

Lameness examination with flexion test and treatments have been performed by a veterinarian, and the results are shown in Fig. 2A.

Samples of synovial fluid and serum were taken from the horse at the time points indicated in Figs. 2B and C. Analysis of COMP epitope concentrations in synovial fluid and serum was carried out as in WO2017216289, and is shown in Fig. 2B and C, respectively. The presence of the COMP epitope indicates ongoing inflammation and tissue damage in the joint.

As seen in Fig. 2A, treatment with low concentration of a local anaesthetic together with glucose caused clinical recovery, associated with decrease of COMP levels to normal physiological levels as seen in Figs. 2B and 2C.

### Example 5 - Horse number 2.

Horse number 2 is a standardbred trotter, stallion, born 2012. The horse had had severe lameness since august 2016, associated with osteoarthritis in his carpal joints. He was treated with 10 ml glucose/mepivacaine (according to Example 1) intra-articular injections several times in the carpal joints since August 2016 and was considered as having failed this treatment.

He received two intra-articular 10 ml intra-articular injections with glucose/mepivacaine/sildenafil (as in Example 3) in the same joints on June 12th and 25^{th}, 2018 and was in October 2018 fully recovered with no lameness recorded at clinical examination. The stallion has been competing in trotting races in August and September 2018, and was then placed 2, 5 and 2 in the races, which indicated full recovery.

Lameness examination with flexion test and treatments have been performed by a veterinarian, and the results are shown in Fig. 3A.

Analysis of COMP epitope concentrations in synovial fluid and serum was carried out as in Example 4, and are shown in Figs. 3A and B, respectively.

As seen in Fig. 3A, addition of sildenafil to the treatment caused clinical recovery, associated with decrease of COMP levels to normal physiological levels as seen in Figs. 3B and 3C.

### Example 6 - Horse number 3

Horse number 3 is standardbred trotter, mare, born 2012. She received her first lameness in both carpal joints as a three-years old. She was treated 17 times with glucose/mepivacaine intra-articular in both carpal joints since then and was considered as having failed this treatment.

In May 2018 she received intra-articular injection with glucose/mepivacaine/sildenafil as in Example 5 in both carpal joints. The horse is now non-lame at lameness examination in May and September 2018 and is competing regularly since then.

Analysis of COMP epitope concentrations in synovial fluid and serum was carried out as in Example 4. Data from clinical lameness examination and COMP levels are shown in Figs. 4A-C. As seen in Fig. 4A, addition of sildenafil to the treatment caused clinical recovery, associated with decreased levels of COMP, as seen in Figs. 4B and 4C.

### Example 7 - Horse number 4

Horse number 4 is a pony, gelding, with severe lameness in the right coffin joint associated with osteoarthritis diagnosed with Magnetic Resonance Imagining (MRI). The horse has been treated with intra-articular injections of corticosteroids since April 2018 three times with no reduction of lameness grade. In June and July 2018, the horse was treated with glucose/mepivacaine/sildenafil (GMS) and is after that non-lame (figure 5A). COMP neo-epitope concentrations in synovial fluid and serum was clearly reduced after the first treatment with GMS (figure 5B and C). Analysis of COMP epitope concentrations in synovial fluid and serum was carried out as in Example 4.

While the invention has been described with reference to specific exemplary embodiments, the description is in general only intended to illustrate the inventive concept and should not be taken as limiting the scope of the invention. The invention is generally defined by the claims.

## Claims

1. A phosphodiesterase type 5 (PDE5) inhibitor for use in the treatment of osteoarthritis in humans, horse, dog or cat, where the PDE5 inhibitor is administered together with a local anaesthetic and glucose.

2. The PDE5 inhibitor for use according to claim 1, where the PDE5 inhibitor is selected from the group consisting of sildenafil, avanafil, lodenafil, mirodenafil, tadalafil, vardenafil and udenafil, hongdenafil, aildenafil, homosildenafil, nitrosoprodenafil, sulfoaildenafil and zaprinast.

3. The PDE5 inhibitor for use according to claim 1 or 2, where the local anaesthetic is of the amino amide type.

4. The PDE5 inhibitor for use according to any one of claims 1 to 3 for treatment in horse.

5. The PDE5 inhibitor for use according to any one of claims 1 to 4 where the PDE5 inhibitor is injected into the affected joint.

6. The PDE5 inhibitor for use according to any one of claims 1 to 5 where a dose of PDE5 inhibitor, amino amide type local anastatic and glucose is administered once, twice or three times.

7. The PDE5 inhibitor for use according to claim 6 where the dose of PDE5 inhibitor, amino amide type local anastatic and glucose is administered two times.

8. The PDE5 inhibitor for use according to any one of claims 1 to 7 where the dose of PDE5 inhibitor is from 0.01 mg to 0.1 mg and where the dose is administered at least once by intraarticular injection

9. The PDE5 inhibitor for use according to any one of claims 1 to 8 where the local anaesthetic is mepivacaine.

10. The PDE5 inhibitor for use according to claim 9 where the dose of glucose is from 100 mg to 1000 mg and the dose of mepivacaine is from 10 mg to 200 mg.

11. A pharmaceutical composition comprising a PDE5 inhibitor, glucose and a local anastatic.

12. The pharmaceutical composition according to claim 11 where the local anastatic is of the amino amide type.

13. The pharmaceutical according to claim 11 or 12 which is an injection solution.

14. The pharmaceutical according to any one of claims 11 to 13 for use in the treatment of osteoarthritis in humans, horse, dog or cat.
